(19) **Europäisches Patentamt / European Patent Office / Office européen des brevets**

(11) **EP 2 772 224 B1**

(12) **EUROPEAN PATENT SPECIFICATION**

(45) Date of publication and mention of the grant of the patent:
**31.08.2022 Bulletin 2022/35**

(21) Application number: **12844223.3**

(22) Date of filing: **24.10.2012**

(51) International Patent Classification (IPC):
**A61C 19/04** (2006.01)   **A23G 4/00** (2006.01)
**A61B 5/22** (2006.01)

(52) Cooperative Patent Classification (CPC):
**A61B 5/228; A61C 19/04;** A61C 2201/002

(86) International application number:
**PCT/JP2012/006801**

(87) International publication number:
**WO 2013/061583 (02.05.2013 Gazette 2013/18)**

(54) **ASSESSING XYLITOL CHEWING GUM MASTICATION FORCE**

BEURTEILUNG DER KAUBARKEIT EINES XYLITOL-KAUGUMMIS

ÉVALUATION DE LA FORCE DE MASTICATION D'UNE GOMME À MÂCHER AU XYLITOL

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **26.10.2011 JP 2011235058**

(43) Date of publication of application:
**03.09.2014 Bulletin 2014/36**

(73) Proprietor: **Lotte Co., Ltd.**
**Tokyo 160-0023 (JP)**

(72) Inventors:
• **SATO, Makoto**
  **Saitama-shi**
  **Saitama 336-8601 (JP)**
• **TOKUMOTO, Takumi**
  **Saitama-shi**
  **Saitama 336-8601 (JP)**
• **HIRAOKA, Yasutaka**
  **Saitama-shi**
  **Saitama 336-8601 (JP)**
• **SASAKI, Ryota**
  **Tokyo 160-0023 (JP)**
• **SUGITA, Daigo**
  **Tokyo**
  **1600023 (JP)**
• **MINAKUCHI, Shunsuke**
  **Tokyo 113-8510 (JP)**
• **UCHIDA, Tatsuro**
  **Tokyo 113-8510 (JP)**

• **KANAZAWA, Manabu**
  **Tokyo 113-8510 (JP)**
• **HAMA, Yohei**
  **Tokyo 113-8510 (JP)**

(74) Representative: **Hartz, Nikolai**
**Wächtershäuser & Hartz**
**Patentanwaltspartnerschaft mbB**
**Weinstrasse 8**
**80333 München (DE)**

(56) References cited:
**WO-A1-2008/111411   WO-A1-2011/040031**
**US-A1- 2012 253 232**

• **Y. KOMAGAMINE ET AL: "Association between masticatory performance using a colour-changeable chewing gum and jaw movement", JOURNAL OF ORAL REHABILITATION, vol. 38, no. 8, 12 August 2011 (2011-08-12), pages 555-563, XP055171432, ISSN: 0305-182X, DOI: 10.1111/j.1365-2842.2011.02204.x**
• **KAMIYAMA MIHO ET AL.: 'Validity and reliability of a Self-Implementable method to evaluate masticatory performance: Use of color-changeable chewing gum and a color scale' JOURNAL OF PROSTHODONTIC RESEARCH vol. 54, 2010, pages 24 - 28, XP026784886**

- **STOTT G. J. ET AL.: 'A simple and reliable method for estimating haemoglobin.' BULLETIN OF THE WORLD HEALTH ORGANIZATION vol. 73, no. 3, 1995, pages 369 - 373, XP002358254**

**Description**

**Technical Field**

The present invention relates to a method for creating a color scale for a xylitol gum for assessing the masticatory force.

**Background Art**

Various methods for directly assessing the masticatory force have been developed and reported so far. Methods for directly assessing the masticatory force are roughly divided into a subjective method in which an edible food product is evaluated by questioning a subject, and a method in which the state of a sample that has actually been masticated is objectively evaluated. Of these methods, the latter method, which does not require a subjective judgment of a subject, is capable of evaluating the masticatory force more quantitatively. On the other hand, this method involves complex operations, requires special equipment, and so forth, and therefore cannot be readily performed in the field of dental diagnosis as well as in routine settings.

In order to solve the aforementioned problems, the present applicants have developed a color-changeable chewing gum, which changes its color as mastication proceeds, and actually put it on the market under the name of Xylitol gum for assessing the masticatory force (trade name). This color-changeable chewing gum changes its color from green to pink as mastication proceeds, and the masticatory force can be easily assessed by evaluating the color after mastication. Also, from the material standpoint, a chewing gum exhibits stable properties, and mass production of uniform products is possible. Further, a chewing gum has many advantages as a measurement sample, for example, it is orally taken in everyday life and is a food having a uniform texture.

Further, the present applicants have also attempted to measure the masticatory force using this color-changeable chewing gum. For example, Non Patent Literatures 4 and 2 have reported that there is relevance between the results obtained by the method for evaluating the masticatory force using the color-changeable chewing gum and those obtained with the conventional sieve method, and further, the degree of color change in this color-changeable chewing gum progresses as mastication proceeds. Further, in order to evaluate the masticatory force more simply, in Non Patent Literature 1, an experimental color scale is created and inter-rater reliability of a method for comparing the masticatory force, which is conducted by comparing the color change in the color-changeable chewing gum with the color scale, is disclosed. That is, it is shown that when a plurality of people keep the score by visually observing the gum after mastication using the color scale created in Non Patent Literature 1, the same scores are obtained, regardless of who keeps the score. This result has demonstrated that the evaluation of the color of the gum can be performed with high reproducibility with this color scale. Moreover, Non Patent Literature 3 looks into the clinical evaluation of the color-changeable chewing gum.

However, the color scale used in Non Patent Literature 1 is created based on visual observation of the color tone of a gum after mastication, but not on a quantitative study of the color change. In addition, this color scale is created based on the mastication results by randomly selected dentists. In view of the above, in order to create a color scale that enables a more objective and quantitative assessment, there is a need for detailed studies on the color change in a gum during mastication by a person having an average masticatory force from immediately after the start of mastication.

Furthermore, in Patent Literature 1, the present applicant has reported a method for creating a color scale, aiming at a color scale that enables a more objective and quantitative assessment. However, there is still a need for the creation of a color scale that more closely conforms to the actual state of the color change in a gum.

Patent Literature 2 describes a method for creating a color scale for determination of masticatory performance. It teaches a quadratic curve/function as a model of the regression equation representing a relationship between a color difference $\Delta E$ and the number of times of mastication. PTL 2 does not disclose a logistic curve, let alone a 4-parameter logistic curve, as a model of the regression equation representing a relationship between the color difference and the number of times of mastication.

**Citation List**

**Patent Literature**

PTL 1: Japanese Patent Application Laid-Open No. 2011-72559
PTL 2: WO 2011/040031 A1

**Non Patent Literature**

NPL 1: Fujinami et al.: "Reliability of Evaluation Method of Masticatory Ability by Color-Changeable Gum Using Color Scale," Journal of Japanese Society for Masticatory Science and Health Promotion, 18(2), p. 173 to 174, 2008
NPL 2: Hirano et al.: "A Study on Measurement of Masticatory Ability Using a Color-changeable Chewing Gum with a New Coloring Reaction", The Journal of The Japan Prosthodontic Society, 46, pp. 103 to 109, 2002
NPL 3: Clinical Evaluation of Masticatory Ability - Seeking for Further Objectivity- "Application of a Color-Changeable Chewing Gum," Journal of Japanese Society for Masticatory Science and Health Promotion, 12(2), pp. 92 to 93, 2003
NPL 4: Hayakawa et al.: "A Simple Method for Evaluating Masticatory Performance Using a Color-Changeable Chewing Gum," The International Journal of Prosthodontics, 11(2): pp. 173 to 176, 1998

**Summary of Invention**

**Technical Problem**

An object of the present research is to create a color scale that can be used, for the evaluation of the masticatory force by a color-changeable chewing gum, as more objective and quantitative assessment criteria that more closely conform to the actual state of the color change in the gum.

**Solution to Problem**

In light of the aforementioned problem, the present invention relates to a method for creating a color scale for assessing the number of times of mastication in a subject based on a color presented by a gum after mastication using a xylitol gum for assessing the masticatory force, comprising a first step of having a plurality of subjects masticate a xylitol gum for assessing the masticatory force a certain number of times, a second step of obtaining a regression equation representing a correlation between a coordinate value of a color presented by the gum after mastication in a specific color space and a color difference in the colors presented by the gum before and the gum after mastication in the color space, a third step of obtaining a regression equation representing a correlation between the color difference and the number of times of mastication, wherein a model of the regression equation is a 4-parameter logistic curve, and a fourth step of determining an average number of times of mastication with respect to a color presented by the gum after mastication using the regression equations obtained in the second and third steps.

Specifically, a regression equation obtained by using a 4-parameter logistic curve as a model and more closely conformed to the actual state of the mastication-induced color change in the "xylitol gum for assessing the masticatory force" was successfully obtained by using a regression equation representing the correlation between the color difference and the number of times of mastication. Further, by creating a color scale using this regression equation, it became possible to assess the masticatory ability more accurately in a manner more closely conforming to the actual state of the color change in a gum.

**Advantageous Effects of Invention**

According to the method of the present invention, a color scale for assessing the masticatory force more objectively and quantitatively than the conventional method can be created. In particular, by creating a color scale using the new regression equation, the masticatory ability can be more accurately measured and the assessment of masticatory ability using the "xylitol gum for assessing the masticatory force" can be easily carried out in clinical settings.

**Brief Description of Drawings**

Fig. 1 is a graph illustrating the correlation between the number of times of mastication and L*a*b* in one Example of the present invention.
Fig. 2 is a graph illustrating the correlation between the number of times of mastication and L*a*b* in one Example of the present invention.
Fig. 3 is a graph illustrating the correlation between the number of times of mastication and L*a*b* in one Example of the present invention.
Fig. 4 is a graph illustrating the correlation between the number of times of mastication and L*a*b* in one Example of the present invention.

Fig. 5 is a graph illustrating the correlation between ∆E and the number of times of mastication in one Example of the present invention.

Fig. 6 is a graph illustrating the correlation between ∆E and the number of times of mastication in one Example of the present invention.

Fig. 7 is a graph illustrating the inter-subject reliability of the color change in a gum in one Example of the present invention.

Fig. 8 is a graph illustrating the correlation obtained by a regression equation of the color adopted in the color scale (∆E) and the corresponding number of times of mastication in one Example of the present invention.

## Description of Embodiments

The following points are pointed out for the method for creating a color scale of Patent Literature 1: That is, 1) the regression equation model is a quadratic function, which is different from the actual state of the color change in a gum, 2) inter-subject reliability (reproducibility) of the color change in a gum is not studied, and 3) uniformity of the tendency of color change in a gum among subjects is not studied.

In light of the foregoing, the following study was carried out in the present invention in an attempt to compensate for the shortcomings of Patent Literature 1. That is, the advantages of the color scale of the invention of the present application will be described in detail in connection with the aforementioned three points.

1') About regression equation model

A 4-parameter logistic curve was used as a model for the new regression equation. The regression equation of Patent Literature 1 is a quadratic function, according to which as the number of times of mastication approaches ∞ (number of times of mastication → ∞), ∆E approaches ∞ (∆E → ∞). There is an apparent contradiction in ∆E = ∞. In a truly strict sense, no upper limit may be imposed on ∆E; however, insofar as a gum is used, it was considered appropriate to assume that ∆E has a certain upper limit. In fact, as a result of having a plurality of people masticate gums 500 times and 600 times, the value of ∆E was found to approach a nearly constant value. Among those values, the maximum value ∆Emax = 73.2 was adopted as the maximum value of ∆E, and in the new regression equation obtained by using a 4-parameter logistic curve as a model, the upper asymptote of ∆E was set at 73.2. As a consequence, a regression equation that more closely conformed to the actual state of the color change in a gum was obtained.

Regression equation for the color scale of the invention of the present application

[Expression 1]

$$\Delta E = k_1 + \frac{k_2 - k_1}{1 + e^{k_3(N - k_4)}}$$

$$k_1 = 73.2$$
$$k_2 = -28478259.6$$
$$k_3 = 0.01$$
$$k_4 = -1352.1$$

2') Inter-subject reliability (reproducibility) of the color change in a gum

In the experiment of Patent Literature 1, each of the subjects (61 people) masticated 20, 40, 60, 80, 120, and 160 times, with the measurement taken once for each number of times of mastication. The problem is that the measurement was taken "once." A measurement value consists of the "true value of a subject" and a "measurement error" (measurement value = true value + measurement error). The inter-subject reliability of the color change in a gum is the proportion of the true value measured in the measurement value. In other words, the inter-subject reliability of the color change in a gum indicates the degree of influence of the measurement error, or the reproducibility of the measurement value. For example, the inter-subject reliability of the color change in a gum is an index of the degree of fluctuation of the value of ∆E when a subject masticated 20 times, or an index of the degree of reproducibility of the value of ∆E. With respect to the measurement results of individual subjects in Patent Literature 1, it was unknown how accurately the true value (true masticatory ability) of each subject was measured. When true values are (probably) not correctly measured, a correct regression equation cannot be created.

In the experiments of the invention of the present application, with respect to each of the subjects (10 people), five measurements were taken for each number of times of mastication. By doing so, it was made possible to study the inter-subject reliability of the color change in a gum. Although it turned out that there was no major problem with taking measurement only "once," a regression equation was drawn from the experimental data of the invention of the present application, for which five measurements were taken to create a more accurate regression equation. In the experiments of the present application, 100 times of mastication as well as 200 times of mastication were also performed only to create a more precise regression equation, although this was not necessarily needed.

3') Uniformity of the tendency of the color change in a gum among subjects

The phrase "to create a color scale" refers to create a color scale that corresponds to the "tendency of the color change in a gum corresponding to the number of times of mastication" that is common to all of the subjects. That is, the premise is that the tendency of the color change in a gum corresponding to the number of times of mastication is "common to all of the subjects."

For example, if it is supposed that there are various types of subjects, e.g., a type of subjects exhibiting a great color change in the first half of the mastication but growing tired from the middle of mastication (a boost-start type), a type of subjects exhibiting a sudden color change in the middle of mastication, and a type of subjects who catch up in the last half of mastication (a catch-up type), the very act of creating a color scale becomes impossible. This point has not been considered in the experiment of Patent Literature 1.

In order to study the point mentioned above, the following analysis was performed.

First of all, with respect to 10 subjects, a regression equation was drawn for each subject using a 4-parameter logistic curve as a model. Using the resulting equation, the number of times of mastication necessary for each subject to achieve a certain color difference $\Delta E (= 1$ to $73)$ $(N\Delta E)$ was calculated. This $N\Delta E$ can be regarded as an evaluation value of masticatory ability. The ratio between $N\Delta E$s (i.e., $N\Delta E$ of Subject 1 / $N\Delta E$ of Subject 2, and so forth) is calculated for each $\Delta E$ based on Subject 1. At $\Delta E = 20$ or above, the ratio of $N\Delta E$ among subjects reached a nearly constant value. This indicates that at $\Delta E = 20$ or above, the relative masticatory ability of a subject can be constantly measured, irrespective of the progress in mastication. That is, irrespective of the number of times of mastication, the relationship between the masticatory ability of one subject and that of another subject can be constantly measured (for example, the relationship between the masticatory ability of subject 1 and that of subject 2 is such that one's masticatory ability is 1.5 times that of the other at 60 times, at 100 times, and even at 160 times of mastication). Namely, this suggests that there is neither a subject of the aforementioned boost-start type nor a subject of the aforementioned catch-up type, and the color change occurs with the same tendency in all of the subjects.

Having confirmed the above findings, a new regression equation was drawn from all the data obtained from the experiments of the present application.

**Examples**

An example of the method for creating the color scale that was created in the present Examples will be described below. It should be noted that the present invention is not limited to the following configurations with respect to the subject, the number of subjects, the number of times of mastication, the method for measuring color, and so forth.

According to the present invention, the masticatory force means the ability to crush and blend food and mix the food with saliva. According to the present invention, the masticatory force can be expressed as follows. That is, with respect to the degree to which a chewing gum is crushed, blended, and then mixed with saliva after being masticated a certain number of times, find out the number of times of mastication required for subjects to crush, blend, and then mix chewing gums with saliva to the same degree, and compare the resulting numbers of times of mastication among the subjects.

1. Xylitol gum for assessing the masticatory force

The chewing gums used are the same as those actually on the market, which are the type of gums that have a plate-like form $(36 \times 20 \times 5$ mm, 3.0 g) that contain, as the main components, a gum base, citric acid, xylitol, and red, yellow, and blue dyes, and the like. Because the gum has a low pH due to the presence of citric acid, and the red dye is made with a synthetic colorant that does not develop color in the acidic region, the gum presents a yellowish green color due to the presence of yellow and blue dyes before mastication. As the gum is mixed with saliva as mastication proceeds, the yellow and blue dyes are eluted from the gum base. At the same time, elution of citric acid into saliva elevates the pH inside the gum, resulting in the red dye developing a red color. As a result, the gum turns from yellowish green color to red color. It should be noted that as long as the conditions that the color change is caused by mastication are met, no limitation is imposed on the gum component and colorant component other than those described above, and any color-changeable chewing gum can be used. The formulation of the xylitol gum for assessing the masticatory force used in the present Examples is shown in Table 1.

[Table 1]

Table 1

| Gum base | 23% |
|---|---|
| Sugar alcohols (xylitol and maltitol) | 75% |
| Fragrance, etc. | 2% |
| Citric acid | 0.1% |
| Food red No. 3 (in the gum) | 0.01% |
| Food yellow No. 4 (in the gum) | 0.01% |
| Food blue No. 1 (in the gum) | 0.001% |

2. Reliability of the gum for assessing the masticatory force

As the currently employed objective method for evaluating the masticatory ability, a sieve method, a method using gummy jelly, a method using a wax cube, and the like are available. However, these methods involve complex measurement processes, and moreover, require specialists as well as special equipment to perform a test. Therefore, it is difficult to apply these methods to a wide range of areas such as home and nursing facilities. In light of this, a gum that changes its color as mastication proceeds (xylitol gum for assessing the masticatory force (trade name)) was developed as a sample enabling simple evaluation of masticatory ability, and it was reported that it was possible to express the masticatory ability as a numerical value by measuring the color by a colorimeter. In order to establish a new test method, its validity and reliability must be studied. Validity has a property of indicating how accurately the object to be measured is measured, and reliability has a property of indicating reproducibility of the results obtained from a plurality of measurements, reproducibility of the results obtained from a measurement carried out by a plurality of examiners, and so forth. An object of the present research is to study the inter-subject reliability of the measurement results obtained with a xylitol gum for assessing the masticatory force, that is, the reproducibility of the color change in the gum when the same subject masticates the gum the same number of times.

(Method)

Ten people having healthy toothed jaws (aged 26 to 30 years, an average age was 27.7 years old) were instructed to masticate the xylitol gums for assessing the masticatory force 20, 40, 60, 80, 100, 120, 160, and 200 times, five trials for each number of times of mastication. They were instructed to take a long enough time interval between trials, and as the conditions of mastication, perform mastication at the preferred chewing side, one masticatory movement per second, until the intercuspal position was reached. On completion of predetermined numbers of times of mastication, the gums were taken out and immediately wrapped in a polyethylene film, and pressed to a thickness of 1.5 mm using a glass slab. Using a colorimeter (CR-13, Konica Minolta, Inc.), the $L^*$, $a^*$, and $b^*$ values were measured at a total of five sites, which were the center and 3 mm from the center in the vertical and horizontal directions, and from the values thus obtained, the color difference $\Delta E$ from the samples before mastication was obtained.

(Results and discussion)

In the present research, Shrout ICC (1,1) was used as an index of inter-subject reliability. ICC (1,1) and the lower limit of 95% confidence interval at the numbers of times of mastication of 20, 40, 60, 80, 100, 120, 160, and 200 are shown in Table 2. Generally, it is determined that reliability is substantial when ICC is 0.61 to 0.80, and reliability is almost perfect when ICC is 0.81 to 1.00. According to the present experiment, ICC (1,1) was 0.8 or above and the lower limit of 95% confidence interval was 0.6 or above at the numbers of times of mastication of 80, 100, 120, and 160, suggesting that adequate reliability was demonstrated at these numbers of times of mastication.

[Table 2]

Table 2

| Number of times of mastication | ICC (1,1) | Lower limit of 95% confidence interval |
|---|---|---|
| 20 | 0.48 | 0.20 |
| 40 | 0.66 | 0.40 |

(continued)

| Number of times of mastication | ICC (1,1) | Lower limit of 95% confidence interval |
| --- | --- | --- |
| 60 | 0.79 | 0.58 |
| 80 | 0.84 | 0.67 |
| 100 | 0.81 | 0.62 |
| 120 | 0.89 | 0.76 |
| 160 | 0.83 | 0.65 |
| 200 | 0.72 | 0.48 |

3. Mechanism of color change in the xylitol gum for assessing mastication

The mastication-induced color change in the xylitol gum for assessing the masticatory force occurs by the following mechanism. The gum internally contains a green dye (blue and yellow dyes) and a red dye. The green dye presents a green color before mastication, which flows out into saliva by mastication, resulting in decoloration. Also, the red dye has a property of remaining nearly colorless under acidic conditions but strongly developing the color under neutral and alkaline conditions. Due to the presence of citric acid in the gum, the red dye remains nearly colorless before mastication. As citric acid flows out by mastication, the inside of the gum becomes neutral, resulting in the development of an intense red color. By these mechanisms, the gum changes its color from green color before mastication to red color after mastication. This time, a study was carried out with an intention to study the color change in the dyes (green and red dyes) contained in the gum along with mastication to elucidate the mechanism of color change in the xylitol gum for assessing the masticatory force.

(Method)

The subjects were four people with healthy toothed jaws (four males, an average age was 21.5 years old, aged 21 to 23 years). The subjects were instructed to masticate (1) the xylitol gums for assessing the masticatory force, (2) gums containing only green dyes, (3) gums containing only red dyes, and (4) gums containing no dye, predetermined numbers of times (20, 40, 60, 80, 100, 120, 160, and 200 times). All the predetermined numbers of times of mastication were performed for one of (1) to (4) in a single day. The subjects were instructed to avoid mastication immediately after meal and perform mastication at the habitual chewing side at a pace of one masticatory movement per second using a metronome after rinsing the mouth for 30 seconds with tap water. Also, in order to prevent fatigue due to mastication of the gums and habituation to mastication, a recess of two hours or longer was given when the total number of times of mastication exceeded 260. After masticating the gums predetermined numbers of times, the gums were immediately wrapped in a polyethylene sheet and pressed to a thickness of 1.5 mm using a glass slab. Using a colorimeter (CR-13, Konica Minolta, Inc.), $L^*$, $a^*$, and $b^*$ were measured at a total of five sites, which were the center and 3 mm from the center in the vertical and horizontal directions, and an average value of the five sites was used as the measurement value. The number of times of mastication-$L^*$ characteristics curve, number of times of mastication-$a^*$ characteristics curve, and number of times of mastication-$b^*$ characteristics curve were obtained from the gums of (1) to (4), and the color change along with mastication was studied. The subjects were informed of the content of the research and the experiments were performed with consent.

(Results and discussion)

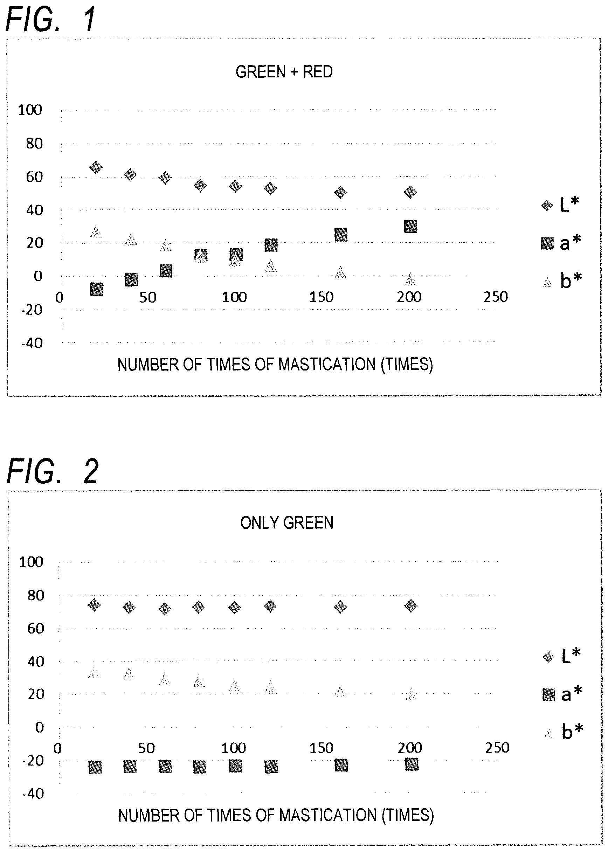

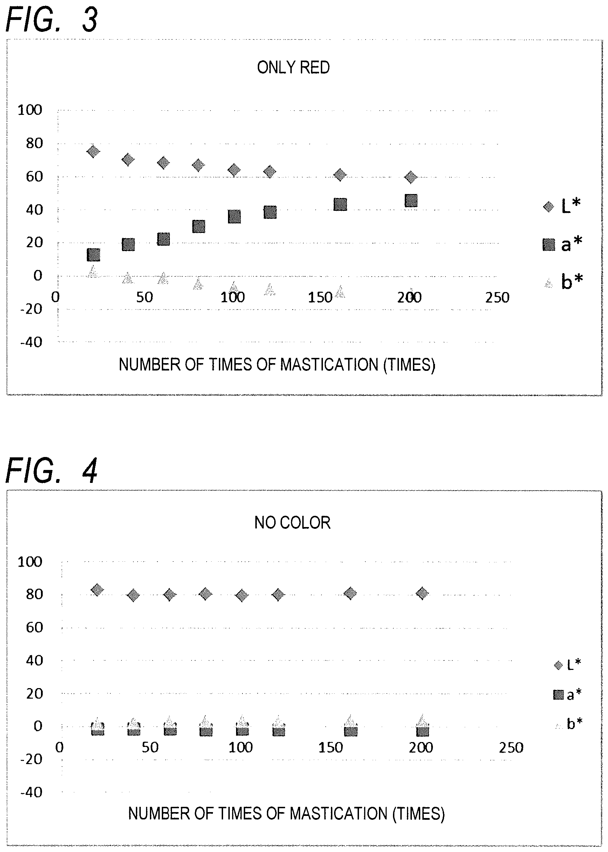

The results thus obtained are each shown in Figs. 1 to 4.
In the xylitol gums for assessing the masticatory force, $L^*$ decreased, $a^*$ increased, and $b^*$ decreased as mastication proceeded, resulting in the gums turning from dark yellowish green color to dark reddish purple color (Fig. 1).
In the gums containing only green dyes, the values of $L^*$ and $a^*$ stayed constant, while $b^*$ decreased as mastication proceeded, resulting in the gums turning from clear yellowish green color to clear light green color (Fig. 2). Based on the above observation, it was speculated that the decoloration of yellow dyes might have outpaced the decoloration of blue dyes.
In the gums containing only red dyes, $L^*$ and $b^*$ decreased, while $a^*$ increased as mastication proceeded (particularly, $a^*$ drastically increased from 60 times to 160 times of mastication), resulting in the gums turning from light pink color to clear reddish purple color (Fig. 3). Based on the observation of not only an increase in $a^*$, but also a decrease in $b^*$, it was speculated that the color development of red dyes also involved the components of blue dyes.

In the gums containing no dye, no color change was observed along with the progress in mastication (Fig. 4) .

Further, the intensity of color change in each gum was investigated by obtaining the color difference ΔE of each gum before and after mastication. The average of four subjects was used for graphing.

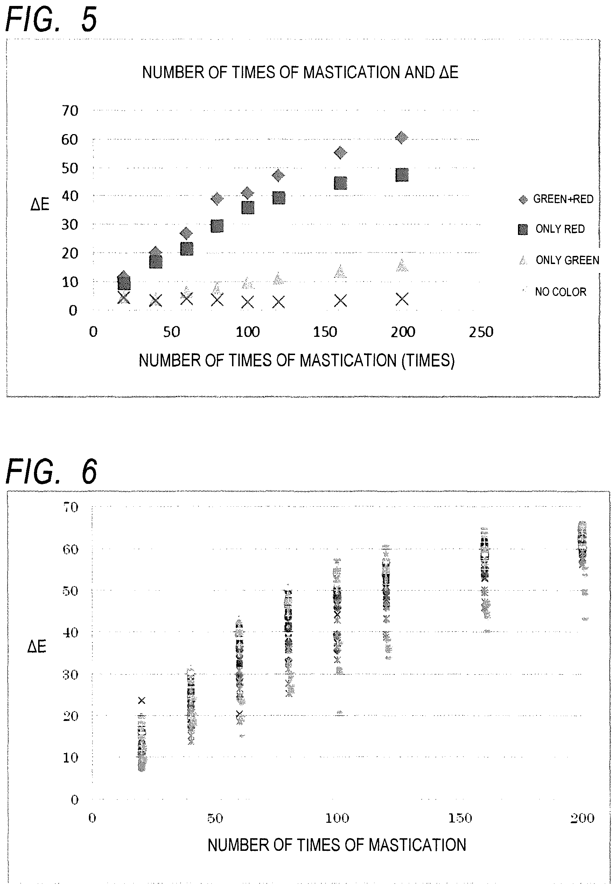

The results thus obtained are shown in Fig. 5. These results suggested that red dyes had a greater impact on color change than did green dyes.

4. Development of a color scale for a color-changeable gum in units of ratio scale

As the currently employed objective method for evaluating the masticatory ability, a sieve method, a method using gummy jelly, a method using a wax cube, and the like are available. However, these methods involve complex measurement processes, and moreover, require specialists as well as special equipment to perform the test itself. Therefore, it is difficult to apply these methods to a wide range of areas such as home and nursing facilities. In light of this, the present inventors developed a gum that changes its color as mastication proceeds (xylitol gum for assessing the masticatory force) as a sample enabling simple evaluation of masticatory ability. The present inventors have already reported that the masticatory ability can be expressed as a numerical value by measuring the color by a colorimeter, and further, developed a color scale that enables assessment of masticatory ability without using a colorimeter. The evaluation of masticatory ability in a wide range of areas was made possible by the use of this color scale. However, the results obtained with this color scale are on an ordinal scale, and quantitative evaluation was not possible. In the present invention, experiments were carried out with the aim of developing a new color scale that can provide results on a ratio scale.

(Method)

The subjects were 10 people having healthy toothed jaws (aged 26 to 30 years, an average age was 27.7 years old), and measurements were taken at the numbers of times of mastication of 20, 40, 60, 80, 100, 120, 160, and 200, five trials for each number of times of mastication. As the conditions of mastication, they were instructed to perform mastication at the preferred chewing side, one masticatory movement per second, until the intercuspal position was reached. On completion of predetermined numbers of times of mastication, the gums were taken out and immediately wrapped in a polyethylene film, and pressed to a thickness of 1.5 mm using a glass slab. Using a colorimeter (CR-13, Konica Minolta, Inc.), the L*, a*, and b* values were measured at a total of five sites, which were the center and 3 mm from the center in the vertical and horizontal directions, and from the values thus obtained, the color difference ΔE from the samples before mastication was obtained. After confirming the uniformity of the tendency of color change among the subjects, a regression equation, in which the color difference ΔE is a dependent variable and the number of times of mastication N is an explanatory variable, (hereinbelow, regression equation 1) was obtained. As the software for regression analysis, JMP8 was used, and as a model of the regression equation, a 4-parameter logistic curve was adopted. Aside from this regression equation, a regression equation for ΔE and L*, a*, and b* (hereinbelow, regression equation 2) was also obtained from the same data, and using these equations, a new color scale that enables quantitative evaluation was created.

(Results)

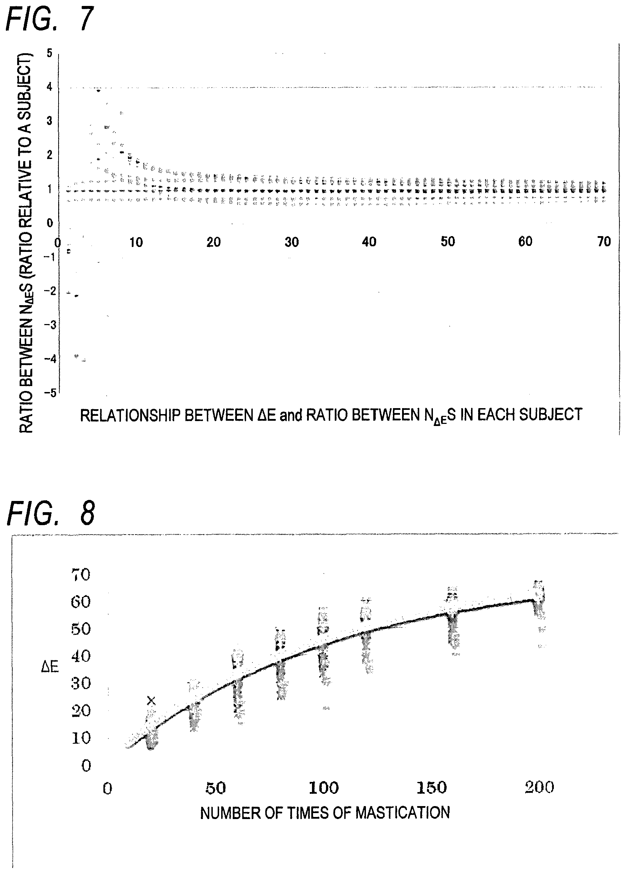

First of all, the uniformity of the tendency of color change among the subjects was studied. A regression equation for ΔE-N was obtained for each subject, and the number of times of mastication necessary for each subject to achieve a certain color difference ΔE ($N_{\Delta E}$) was calculated, and the scale factor among subjects between $N_{\Delta E}$s at each ΔE was calculated. As a result, the calculated scale factor was found to be a nearly constant value, particularly at ΔE of 20 or above in each subject (Fig. 7), suggesting that the color change progressed with the same tendency according to the progress in mastication in a manner independent of the subject. Subsequently, regression analysis was performed using all the data to obtain a regression equation for creation of a color scale (regression equation 1). From the regression equation 2, L*, a*, and b* at each ΔE were obtained and the color of each ΔE was determined. Among the colors thus obtained, appropriate ones were adopted for a color scale, and $N_{\Delta E}$ for the adopted colors was obtained from the regression equation 1, and the result thus obtained was used as the numerical value of the result of each color in the color scale. For simplicity, as well as to minimize errors, the scale representing each color per se was expressed as an integer, in serial order starting with 1, with an interval of 1.

(Conclusion) According to the present experiment, a new color scale in units of the number of times of mastication, which is a ratio scale, was successfully created (Figs. 6 and 8).

(Regression equation 1)

[Expression 2]

$$\Delta E = k_1 + \frac{k_2 - k_1}{1 + e^{k_3(N - k_4)}}$$

$k_1 = 73.2$
$k_2 = -28478259.6$
$k_3 = 0.01$
$k_4 = -1352.1$

(Regression equation 2)

[Expression 3]

L* = -0.3182×ΔE + 72.568
a* = 0.7475×ΔE - 14.591
b* = -0.5862×ΔE + 33.506

Using the color scale thus created, the validity of the method for assessing the masticatory ability using the color scale was studied.

(Method)

The subjects were 10 people having healthy toothed jaws (aged 24 to 31 years, an average age was 27.2 years old), and the subjects masticated the xylitol gums for assessing the masticatory force 100 times. On completion of mastication, the gums were taken out and immediately wrapped in a polyethylene film, and pressed to a thickness of 1.5 mm using a glass slab. Using a colorimeter (CR-13, Konica Minolta, Inc.), the L*, a*, and b* values were measured at a total of five sites, which were the center and 3 mm from the center in the vertical and horizontal directions, and from the values thus obtained, the color difference ΔE from the samples before mastication was obtained. Further, separately, each subject evaluated the sample masticated by oneself based on the color scale, whereby the response values based on the color scale were collected. Subsequently, the Spearman's rank correlation coefficient was obtained with respect to the color difference ΔE and the response values based on the color scale. The significance level was set at 5%, and SPSS17.0 was used as the statistical software.

(Results and discussion)

The results of 100 times of mastication of the gum by each subject were as follows in terms of the response value based on the color scale: That is, two subjects presented a color corresponding to the number of times of mastication of 111.8, four subjects presented a color corresponding to the number of times of mastication of 147.6, three subjects presented a color corresponding to the number of times of mastication of 202.3, and two subjects presented a color corresponding to the number of times of mastication of 323.7. Also, the Spearman's rank correlation coefficient was 0.673 (p = 0.023), verifying that there was a significant correlation between the color difference ΔE and the response value based on the color scale. Based on the fact that the color difference ΔE has come to be recognized as an index of masticatory ability by previous research, it was suggested that the response value based on the color scale, which has a correlation with the color difference ΔE, could also be used as a valid index of masticatory ability.
More objective and quantitative assessment of the masticatory ability of a subject was made possible by using the color scale created in the present experiments.
This application claims the benefit of the priority of Japanese Patent Application No. 2011-235058, filed October 26, 2011, the content of which is hereby incorporated into, and forms part of this application.

**Claims**

1. A method for creating, using a gum that changes its color according to mastication, a color scale for assessing the number of times of mastication in a subject based on a color presented by the gum after mastication, comprising,

a first step of having a plurality of subjects masticate a gum that changes its color according to mastication a certain number of times,
a second step of obtaining a regression equation representing a correlation between a coordinate value of a color presented by the gum after mastication and a color difference between the gum before and the gum after mastication in a color coordinate system,

a third step of obtaining a regression equation representing a correlation between the color difference and the number of times of mastication, wherein a model of the regression equation is a 4-parameter logistic curve, and a fourth step of determining an average number of times of mastication with respect to the color presented by the gum after mastication using the regression equations obtained in the second and third steps.

**2.** The method for creating a color scale according to claim 1, wherein the gum that changes its color according to mastication is a xylitol gum for assessing the masticatory force.

**3.** The method for creating a color scale according to claim 1 or 2, wherein the color coordinate system used is a CIE L*a*b* color coordinate system.

**4.** A method for assessing the masticatory ability, wherein the method comprises creating a color scale according to the method of any one of claims 1 to 3.

**Patentansprüche**

**1.** Verfahren zum Erzeugen einer Farbskala unter Verwendung eines Kaugummis, der seine Farbe in Abhängigkeit vom Kauen ändert, um die Anzahl der Kauvorgänge bei einer Person auf der Grundlage einer Farbe, die der Kaugummi nach dem Kauen aufweist, zu beurteilen, umfassend,

einen ersten Schritt, bei dem eine Vielzahl von Versuchspersonen einen Kaugummi, der seine Farbe in Abhängigkeit vom Kauen ändert, eine bestimmte Anzahl von Malen kauen,
einen zweiten Schritt des Erhaltens einer Regressionsgleichung, die eine Korrelation zwischen einem Koordinatenwert einer Farbe, die von dem Kaugummi nach dem Kauen dargestellt wird, und einem Farbunterschied zwischen dem Kaugummi vor und dem Kaugummi nach dem Kauen in einem Farbkoordinatensystem darstellt,
einen dritten Schritt des Erhaltens einer Regressionsgleichung, die eine Korrelation zwischen dem Farbunterschied und der Anzahl der Male des Kauens darstellt, wobei ein Modell der Regressionsgleichung eine logistische 4-Parameter-Kurve ist, und
einen vierten Schritt des Bestimmens einer durchschnittlichen Anzahl von Malen des Kauens in Bezug auf die Farbe, die der Kaugummi nach dem Kauen aufweist, unter Verwendung der in den zweiten und dritten Schritten erhaltenen Regressionsgleichungen.

**2.** Verfahren zur Erstellung einer Farbskala nach Anspruch 1, wobei der Kaugummi, der seine Farbe in Abhängigkeit vom Kauen ändert, ein Xylit-Kaugummi zur Beurteilung der Kaukraft ist.

**3.** Verfahren zur Erstellung einer Farbskala nach Anspruch 1 oder 2, wobei das verwendete Farbkoordinatensystem ein CIE L*a*b*-Farbkoordinatensystem ist.

**4.** Verfahren zur Beurteilung der Kaufähigkeit, wobei das Verfahren das Erzeugen einer Farbskala gemäß dem Verfahren nach einem der Ansprüche 1 bis 3 umfasst.

**Revendications**

**1.** Procédé pour créer, en utilisant une gomme dont la couleur change en fonction de la mastication, une échelle de couleurs pour déterminer le nombre de mastications chez un sujet sur la base de la couleur que présente la gomme après mastication, comprenant

une première étape de disposition d'une pluralité de sujets qui mastiquent une gomme dont la couleur change en fonction du nombre de mastications,
une deuxième étape d'obtention d'une équation de régression représentant une corrélation entre une valeur de coordonnées de la couleur que présente la gomme après mastication et la différence de couleur entre la gomme avant mastication et la gomme après mastication dans un système de coordonnées de couleur,
une troisième étape d'obtention d'une équation de régression représentant une corrélation entre la différence de couleur et le nombre de mastications, dans laquelle le modèle de l'équation de régression est une courbe logistique à 4 paramètres, et
une quatrième étape de détermination du nombre moyen de mastications par rapport à la couleur que présente la gomme après mastication, utilisant les équations de régression obtenues dans les deuxième et troisième étapes.

2. Procédé pour créer une échelle de couleurs selon la revendication 1, dans lequel la gomme dont la couleur change en fonction de la mastication est une gomme de xylitol pour déterminer la force masticatoire.

3. Procédé pour créer une échelle de couleurs selon la revendication 1 ou 2, dans lequel le système de coordonnées de couleur utilisé est un système de coordonnées de couleur CIE L*a*b*.

4. Procédé pour déterminer la capacité masticatoire, dans lequel le procédé comprend la création d'une échelle de couleurs conformément au procédé selon l'une quelconque des revendications 1 à 3.

## FIG. 1

**GREEN + RED**

NUMBER OF TIMES OF MASTICATION (TIMES)

L*
a*
b*

## FIG. 2

**ONLY GREEN**

NUMBER OF TIMES OF MASTICATION (TIMES)

L*
a*
b*

## FIG. 3

**ONLY RED**

L*
a*
b*

NUMBER OF TIMES OF MASTICATION (TIMES)

## FIG. 4

**NO COLOR**

L*
a*
b*

NUMBER OF TIMES OF MASTICATION (TIMES)

## FIG. 5

NUMBER OF TIMES OF MASTICATION AND ΔE

ΔE

70
60
50
40
30
20
10
0

◆ GREEN+RED
■ ONLY RED
△ ONLY GREEN
× NO COLOR

0      50      100      150      200      250

NUMBER OF TIMES OF MASTICATION (TIMES)

## FIG. 6

ΔE

70
60
50
40
30
20
10
0

0      50      100      150      200

NUMBER OF TIMES OF MASTICATION

# FIG. 7

RELATIONSHIP BETWEEN $\Delta E$ and RATIO BETWEEN $N_{\Delta E}S$ IN EACH SUBJECT

# FIG. 8

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011072559 A
- WO 2011040031 A1
- JP 2011235058 A

### Non-patent literature cited in the description

- **FUJINAMI et al.** Reliability of Evaluation Method of Masticatory Ability by Color-Changeable Gum Using Color Scale. *Journal of Japanese Society for Masticatory Science and Health Promotion,* 2008, vol. 18 (2), 173-174
- **HIRANO et al.** A Study on Measurement of Masticatory Ability Using a Color-changeable Chewing Gum with a New Coloring Reaction. *The Journal of The Japan Prosthodontic Society,* 2002, vol. 46, 103-109
- Clinical Evaluation of Masticatory Ability - Seeking for Further Objectivity- ''Application of a Color-Changeable Chewing Gum. *Journal of Japanese Society for Masticatory Science and Health Promotion,* 2003, vol. 12 (2), 92-93
- **HAYAKAWA et al.** A Simple Method for Evaluating Masticatory Performance Using a Color-Changeable Chewing Gum. *The International Journal of Prosthodontics,* 1998, vol. 11 (2), 173-176